# EUROPEAN PATENT SPECIFICATION

(11) **EP 2 417 454 B1**
(45) Date of publication and mention of the grant of the patent: **25.02.2015**
(21) Application number: 10728616.3
(22) Date of filing: 12.04.2010
(51) Int. Cl.: G01N 33/53, G01N 33/68

(54) **METHOD FOR DIAGNOSING AND/OR TYPING RENAL CELL CARCINOMA**
VERFAHREN ZUR DIAGNOSE UND/ODER TYPISIERUNG EINES NIERENZELLKARZINOMS
PROCÉDÉ DE DIAGNOSTIC ET/OU DE TYPAGE DE L'HYPERNÉPHROME

(30) Priority: 10.04.2009 EP 09157820
(43) Date of publication of application: 15.02.2012
(73) Proprietor: PamGene B.V., 5211 's Hertogenbosch (NL); Stichting Katholieke Universiteit, 6525 EZ Nijmegen (NL)
(72) Inventor: RUIJTENBEEK, Robby, NL-3524 BK Utrecht (NL); HOUKES - VAN KERKHOFF, Elisabeth Paullina Maria, NL-5231 HK 's-Hertogenbosch (NL); OOSTERWIJK, Egbert, NL-6641 LM Beuningen (NL)
(74) Representative: De Clercq, Ann G. Y.
(86) International application number: PCT/EP2010/054766
(87) International publication number: WO 2010/115999

(56) References cited:
- WO-A-2008/049930
- US-A1- 2007 254 295
- LEE HYUN JU ET AL: "Phosphorylation of ERK1/2 and prognosis of clear cell renal cell carcinoma." UROLOGY FEB 2009, vol. 73, no. 2, February 2009 (2009-02), pages 394-399, XP002530853 ISSN: 1527-9995
- MIYATA YASUYOSHI ET AL: "Presence of phosphorylated hepatocyte growth factor receptor/c-Met is associated with tumor progression and survival in patients with conventional renal cell carcinoma." CLINICAL CANCER RESEARCH : AN OFFICIAL JOURNAL OF THE AMERICAN ASSOCIATION FOR CANCER RESEARCH 15 AUG 2006, vol. 12, no. 16, 15 August 2006 (2006-08-15), pages 4876-4881, XP002530854 ISSN: 1078-0432
- LAM JOHN S ET AL: "Protein expression profiles in renal cell carcinoma: staging, prognosis, and patient selection for clinical trials." CLINICAL CANCER RESEARCH : AN OFFICIAL JOURNAL OF THE AMERICAN ASSOCIATION FOR CANCER RESEARCH 15 JAN 2007, vol. 13, no. 2 Pt 2, 15 January 2007 (2007-01-15), pages 703s-708s, XP002530855 ISSN: 1078-0432

### SEQUENCE LISTING

<110> PamGene B.V. Stichting Katholieke Universiteit
<120> METHOD FOR DIAGNOSING AND/OR TYPING RENAL CELL CARCINOMA
<130> PAM-056-PCT
<150> EP 09157820.3
   <151> 2009-04-10
<160> 46
<170> PatentIn version 3.3
<210> 1
   <211> 13
   <212> PRT
   <213> Artificial Sequence
<220>
   <223> Peptide
<400> 1
   Ser Asp Thr Glu Glu Gln Glu Tyr Glu Glu Glu Gln Pro
   1               5                   10
<210> 2
   <211> 13
   <212> PRT
   <213> Artificial Sequence
<220>
   <223> Peptide
<400> 2
   Glu Asp Asp Pro Glu Ala Thr Tyr Thr Thr Ser Gly Gly
   1               5                   10
<210> 3
   <211> 15
   <212> PRT
   <213> Artificial sequence
<220>
   <223> Peptide
<400> 3
   Val Arg Arg Glu Val Gly Asp Tyr Gly Gln Leu His Glu Thr Glu
   1               5                   10                  15
<210> 4
   <211> 13
   <212> PRT
   <213> Artificial Sequence
<220>
   <223> Peptide
<400> 4
   Val Gly Glu Glu Glu His Val Tyr Ser Phe Pro Asn Lys
   1               5                   10
<210> 5
   <211> 13
   <212> PRT
   <213> Artificial Sequence
<220>
   <223> Peptide
<400> 5
   Arg Gln Glu Asp Gly Gly Val Tyr Ser Ser Ser Gly Leu
   1               5                   10
<210> 6
   <211> 13
   <212> PRT
   <213> Artificial Sequence
<220>
   <223> Peptide
<400> 6
   Asp Asp Thr Ser Asp Pro Thr Tyr Thr Ser Ser Leu Gly
   1               5                   10
<210> 7
   <211> 13
   <212> PRT
   <213> Artificial Sequence
<220>
   <223> Peptide
<400> 7
   Asn Glu Asn Thr Glu Asp Gln Tyr Ser Leu Val Glu Asp
   1               5                   10
<210> 8
   <211> 13
   <212> PRT
   <213> Artificial sequence
<220>
   <223> Peptide
<400> 8
   Thr Leu Thr Thr Asn Glu Glu Tyr Leu Asp Leu Ser Gln
   1               5                   10
<210> 9
   <211> 13
   <212> PRT
   <213> Artificial Sequence
<220>
   <223> Peptide
<400> 9
   Glu Gly Glu Glu Asp Thr Glu Tyr Met Thr Pro Ser Ser
   1               5                   10
<210> 10
   <211> 15
   <212> PRT
   <213> Artificial Sequence
<220>
   <223> Peptide
<400> 10
   Pro Asn Glu Gly Asp Asn Asp Tyr Ile Ile Pro Leu Pro Asp Pro
   1               5                   10                  15
<210> 11
   <211> 13
   <212> PRT
   <213> Artificial Sequence
<220>
   <223> Peptide
<400> 11
   Met Glu Ser Ile Asp Asp Tyr Val Asn Val Pro Glu Ser
   1 5 10
<210> 12
   <211> 13
   <212> PRT
   <213> Artificial Sequence
<220>
   <223> Peptide
<400> 12
   Thr Tyr Ile Asp Pro Glu Thr Tyr Glu Asp Pro Asn Arg
   1 5 10
<210> 13
   <211> 13
   <212> PRT
   <213> Artificial Sequence
<220>
   <223> Peptide
<400> 13
   Ser Ala Leu Leu Gly Asp His Tyr Val Gln Leu Pro Ala
   1 5 10
<210> 14
   <211> 13
   <212> PRT
   <213> Artificial Sequence
<220>
   <223> Peptide
<400> 14
   Glu Glu Gly Ala Pro Asp Tyr Glu Asn Leu Gln Glu Leu
   1 5 10
<210> 15
   <211> 13
   <212> PRT
   <213> Artificial Sequence
<220>
   <223> Peptide
<400> 15
   Tyr Gln Ala Glu Glu Asn Thr Tyr Asp Glu Tyr Glu Asn
   1 5 10
<210> 16
   <211> 13
   <212> PRT
   <213> Artificial Sequence
<220>
   <223> Peptide
<400> 16
   Ser Glu His Ala Gln Asp Thr Tyr Leu Val Leu Asp Lys
   1               5                   10
<210> 17
   <211> 13
   <212> PRT
   <213> Artificial Sequence
<220>
   <223> Peptide
<400> 17
   Glu Tyr Glu Asp Glu Asn Leu Tyr Glu Gly Leu Asn Leu
   1               5                   10
<210> 18
   <211> 13
   <212> PRT
   <213> Artificial Sequence
<220>
   <223> Peptide
<400> 18
   Lys Val Asp Asn Glu Asp Ile Tyr Glu Ser Arg His Glu
   1               5                   10
<210> 19
   <211> 13
   <212> PRT
   <213> Artificial Sequence
<220>
   <223> Peptide
<400> 19
   Ala Thr Ser Met Phe Asp Asp Tyr Gln Gly Asp Ser Ser
   1               5                   10
<210> 20
   <211> 13
   <212> PRT
   <213> Artificial Sequence
<220>
   <223> Peptide
<400> 20
   Arg Tyr Ile Glu Asp Glu Asp Tyr Tyr Lys Ala Ser Val
   1 5 10
<210> 21
   <211> 13
   <212> PRT
   <213> Artificial Sequence
<220>
   <223> Peptide
<400> 21
   Val Ser Ser Asp Gly His Glu Tyr Ile Tyr Val Asp Pro
   1 5 10
<210> 22
   <211> 13
   <212> PRT
   <213> Artificial Sequence
<220>
   <223> Peptide
<400> 22
   Cys Gln Leu Gly Gln Arg Ile Tyr Gln Tyr Ile Gln Ser
   1 5 10
<210> 23
   <211> 13
   <212> PRT
   <213> Artificial Sequence
<220>
   <223> Peptide
<400> 23
   Ile Gly Thr Ala Glu Pro Asp Tyr Gly Ala Leu Tyr Glu
   1 5 10
<210> 24
   <211> 13
   <212> PRT
   <213> Artificial Sequence
<220>
   <223> Peptide
<400> 24
   Arg Glu Glu Ala Asp Gly Val Tyr Ala Ala Ser Gly Gly
   1 5 10
<210> 25
   <211> 13
   <212> PRT
   <213> Artificial Sequence
<220>
   <223> Peptide
<400> 25
   Ala Arg Thr Thr Ser Gln Leu Tyr Asp Ala Val Pro Ile
   1               5                   10
<210> 26
   <211> 13
   <212> PRT
   <213> Artificial Sequence
<220>
   <223> Peptide
<400> 26
   Ile Glu Asn Glu Glu Gln Glu Tyr Val Gln Thr Val Lys
   1               5                   10
<210> 27
   <211> 13
   <212> PRT
   <213> Artificial Sequence
<220>
   <223> Peptide
<400> 27
   Lys Lys Asp Thr Glu Thr Val Tyr Ser Glu Val Arg Lys
   1               5                   10
<210> 28
   <211> 13
   <212> PRT
   <213> Artificial Sequence
<220>
   <223> Peptide
<400> 28
   Glu Tyr Glu Pro Glu Thr Val Tyr Glu Val Ala Gly Ala
   1               5                   10
<210> 29
   <211> 13
   <212> PRT
   <213> Artificial Sequence
<220>
   <223> Peptide
<400> 29
   Ser Gly Ala Ser Thr Gly Ile Tyr Glu Ala Leu Glu Leu
   1 5 10
<210> 30
   <211> 13
   <212> PRT
   <213> Artificial Sequence
<220>
   <223> Peptide
<400> 30
   Leu Asp Asp Phe Asp Gly Thr Tyr Glu Thr Gln Gly Gly
   1 5 10
<210> 31
   <211> 13
   <212> PRT
   <213> Artificial Sequence
<220>
   <223> Peptide
<400> 31
   Thr Pro Ser Asp Ser Leu Ile Tyr Asp Asp Gly Leu Ser
   1 5 10
<210> 32
   <211> 13
   <212> PRT
   <213> Artificial Sequence
<220>
   <223> Peptide
<400> 32
   Phe Leu Ser Glu Glu Thr Pro Tyr Ser Tyr Pro Thr Gly
   1 5 10
<210> 33
   <211> 13
   <212> PRT
   <213> Artificial Sequence
<220>
   <223> Peptide
<400> 33
   Asp Glu Asp Cys Tyr Gly Asn Tyr Asp Asn Leu Leu Ser
   1 5 10
<210> 34
   <211> 13
   <212> PRT
   <213> Artificial Sequence
<220>
   <223> Peptide
<400> 34
   Gly Ala Gly Phe Gly Ser Arg Ser Leu Tyr Gly Leu Gly
   1               5                   10
<210> 35
   <211> 13
   <212> PRT
   <213> Artificial Sequence
<220>
   <223> Peptide
<400> 35
   Asp Tyr Asn Ser Val Val Leu Tyr Ser Thr Pro Pro Ile
   1               5                   10
<210> 36
   <211> 13
   <212> PRT
   <213> Artificial Sequence
<220>
   <223> Peptide
<400> 36
   Leu Asp Ile Asp Glu Thr Glu Tyr His Ala Asp Gly Gly
   1               5                   10
<210> 37
   <211> 13
   <212> PRT
   <213> Artificial Sequence
<220>
   <223> Peptide
<400> 37
   Ile Ser Leu Asp Asn Pro Asp Tyr Gln Gln Asp Phe Phe
   1               5                   10
<210> 38
   <211> 13
   <212> PRT
   <213> Artificial Sequence
<220>
   <223> Peptide
<400> 38
   Ala Ile Glu Thr Asp Lys Glu Tyr Tyr Thr Val Lys Asp
   1 5 10
<210> 39
   <211> 13
   <212> PRT
   <213> Artificial Sequence
<220>
   <223> Peptide
<400> 39
   Ala Arg Thr Ala His Tyr Gly Ser Leu Pro Gln Lys Ser
   1 5 10
<210> 40
   <211> 13
   <212> PRT
   <213> Artificial Sequence
<220>
   <223> Peptide
<400> 40
   Arg Pro Pro Ser Ala Glu Leu Tyr Ser Asn Ala Leu Pro
   1 5 10
<210> 41
   <211> 13
   <212> PRT
   <213> Artificial Sequence
<220>
   <223> Peptide
<400> 41
   Glu Glu Ala Pro Glu Asp Leu Tyr Lys Asp Phe Leu Thr
   1 5 10
<210> 42
   <211> 13
   <212> PRT
   <213> Artificial Sequence
<220>
   <223> Peptide
<400> 42
   Asp Ile Tyr Lys Asp Pro Asp Tyr Val Arg Lys Gly Asp
   1 5 10
<210> 43
   <211> 13
   <212> PRT
   <213> Artificial Sequence
<220>
   <223> Peptide
<400> 43
   Thr Val Asp Gly Lys Glu Ile Tyr Asn Thr Ile Arg Arg
   1               5                   10
<210> 44
   <211> 12
   <212> PRT
   <213> Artificial Sequence
<220>
   <223> Peptide
<400> 44
   Glu Ala Ile Tyr Ala Ala Pro Phe Ala Lys Lys Lys
   1               5                   10
<210> 45
   <211> 13
   <212> PRT
   <213> Artificial Sequence
<220>
   <223> Peptide
<400> 45
   Val Ala Asp Ile Asp Gly Gln Tyr Ala Met Thr Arg Ala
   1               5                   10
<210> 46
   <211> 13
   <212> PRT
   <213> Artificial Sequence
<220>
   <223> Peptide
<400> 46
   Tyr Met Ala Pro Tyr Asp Asn Tyr Val Pro Ser Ala Pro
   1               5                   10

## Description

### FIELD OF THE INVENTION

The present invention relates to a method for diagnosing, typing and/or subtyping renal cell carcinoma as well as predicting the response to medication of patients suffering from renal cell carcinoma. More specifically, the present invention provides methods which measure kinase activity by studying phosphorylation levels and profiles in samples of said patients.

### BACKGROUND OF THE INVENTION

Renal cell carcinoma (RCC) is the most common form of kidney cancer and responsible for approximately 80% of the kidney cancer that occur in adults. RCC represents approximately 5% of all cancer deaths and at the time of presentation, over 50% of the patients have already developed locally advanced or metastatic disease with 5-year survival rates of less than 20%. RCC originates in the lining of the proximal renal tubule, the very small tubes in the kidney that filter the blood and remove waste products. Renal Cell Carcinoma is sometimes also referred to as Renal Cell Cancer, Renal Adenocarcinoma or Hypernephroma. Most common causes of RCC are smoking, obesity, high blood pressure, long-term dialysis, Von Hippel-Lindau Syndrome and occupation.

RCC can further be classified in a number of different subtypes including clear cell subtype (from 60 to 75%), chromophobic subtype (about 4%), chromophilic subtype, papillary carcinoma subtype (about 12%), oncocytic subtype (about 4%), medullary carcinoma subtype and collecting duct (Bellini's duct, less than 1%) tumors.

The histological cell types of clear cell nature is found in Von Hippel-Lindau (VHL) disease, hereditary paraganglioma (HP), and familial clear cell renal cancer (FCRC). The histological cell types of papillary nature is found in hereditary leiomyomatosis and renal cancer (HLRCC), and hereditary papillary renal cancinoma (HPRC). Both chromophobe and oncocytoma cell types are found in Birt-Hogg-Dube Syndrome (BHD).

One of the major problems with RCC is the late detection of the disease. Often patients present themselves with symptoms such as hematuria, flank pain and/or an abdominal mass. This is now known as the 'too late triad' because by the time patients present with symptoms, their disease is often advanced beyond a curative stage. In addition, whilst this triad is highly suggestive of RCC, it only occurs in around 15% of the sufferers. Today, the majority of renal tumors are asymptomatic and are detected incidentally on imaging, usually for an unrelated cause.

Initial treatment of RCC is the removal of the tumor through surgery. After surgery a radiation therapy and/or chemotherapy is only efficient in a limited number of cases. Most of the RCC are resistant to radiation therapy and/or chemotherapy. Targeted cancer therapies such as treatments with Sunitinib and Sorafenib, have been reported to improve the outlook for RCC, although they have not yet demonstrated improved survival.

RCC elicits an immune response, which occasionally results in dramatic spontaneous remissions. This has encouraged a strategy of using immunomodulating therapies, such as cancer vaccines and interleukin-2 (IL-2), to reproduce this response. IL-2 has produced "durable remissions" in a small number of patients, but with substantial toxicity. Sutinib (Sutent), sorafenib (Nexavar), and temsirolimus, which are small-molecule inhibitors of protein kinases, have been approved by the FDA for the treatment of advanced renal cell cancer. Sunitinib and sorafenib are inhibitors that interfere with tumor growth by inhibiting angiogenesis as well as tumor cell proliferation.

At present, in the field of RCC diagnosis, it is of great importance to detect the tumor at an early stage and furthermore, at the time of the diagnosis, it is also of great importance to enable a prediction of the most suited treatment of the diagnosed RCC. Especially when treating RCC patients with Sorafininb and Sunitinib an unmet medical need exists for a method predicting the patient's response to these drugs.

Ju et al (Urology, 2009, vol.73, no.2) discloses measurements of the expression of a single phosphorylated protein (HGFR/c-Met) using immunohistochemistry. Therefore Ju et al discloses a direct measurement of the expression of a single phosphorylated protein using antibodies. Yasuyoshi et al (Clinical cancer research, 2006, vol.12, no.16) provides a study wherein the expression of phosphorylated HGFR/c-Met is associated with RCC tumor growth and progression. For this study immunohistochemistry was used to measure the expression of phosphorylated HGFR/c-Met. Both Ju et al and Yasuyoshi et al provide a direct measurement of the expression of a single phosphorylated protein using antibodies. WO 2008/049930 discloses arrays having protein kinase substrates which are used for determining a kinase phosphorylation profiles.

Whereas genetic changes can aid these prognostic efforts and predictions, information about a single or a limited number of molecular markers generally fails to provide satisfactory results for the clinical diagnosis and response prediction of RCC.

There remains a pressing need for methods that provide good clinical diagnosis, typing and subtyping of RCC. These methods would enable the correct classification of RCC preferably at an early stage, and more specifically provide an early prediction of the response of the RCC to specific adjuvant therapies.

The present invention aims at providing methods for diagnosing, typing and/or subtyping RCC. The present invention also aims to provide methods and devices for predicting the response of a patient diagnosed with RCC to a medicament.

### SUMMARY OF THE INVENTION

The present invention provides methods that enable the diagnosis typing and/or subtyping of renal cell carcinoma based on the measurement of the kinase activity of a RCC tumor sample. Preferably, in one embodiment of the present invention, methods are provided wherein the kinase activity is protein kinase activity.

The present invention therefore provides a method for diagnosing renal cell carcinoma in a subject. In a first embodiment of the present invention, the method comprises the steps of:
(a) measuring kinase activity of a kidney lysate sample from said subject, by contacting said sample with at least one protein kinase substrate, thereby providing a phosphorylation profile of said kidney sample; and,
(b) determining from said phosphorylation profile the presence or absence of renal cell carcinoma in said subject, thereby diagnosing renal cell carcinoma in said subject.

In another embodiment according to the present invention, the phosphorylation profile comprises the phosphorylation levels of, preferably one or more, phosphorylation site(s) present in any of the peptide markers as listed in Table 1 with SEQ ID NO 1 to 44.

Another embodiment of the present invention relates to a method for predicting the response of a patient diagnosed with renal cell carcinoma to a medicament, wherein the kinase activity of a sample, obtained from the renal cell carcinoma from said patient, is measured in the presence and in the absence of said medicament and wherein said kinase activity in the presence said medicament is compared to the kinase activity in the absence of said medicament thereby determining the response of said patient to said medicament, wherein said kinase activity measurement provides phosphorylation profiles of said sample in the presence and in the absence of said medicament.

More preferably the present invention relates to a method according to the present invention wherein said medicament is a protein kinase inhibitor such as sorafenib or sunitinib.

The present invention further relates in yet another embodiment to a method for typing or subtyping renal cell carcinoma, comprising the steps of:
(a) measuring the kinase activity of a renal cell carcinoma sample according to the method of the present invention, thereby providing a phosphorylation profile of said renal cell carcinoma sample; and,
(b) determining from said phosphorylation profile the type or subtype of renal cell carcinoma.

These and further aspects and embodiments are described in the following sections and in the claims.

### BRIEF DESCRIPTION OF FIGURES

**Figure 1** provides, as depicted in the examples, a heatmap representation wherein the peptide phosphorylation levels of normal kidney (NK) and tumor kidney (RCC) tissue are compared.
**Figure 2** provides, as depicted in the examples, a graphical representation of the diagnostical value of the set of peptide markers.
**Figure 3** provides, as depicted in the examples, a heatmap representation showing the (sub)typing of RCC using the set of peptide markers.
**Figure 4** provides, as depicted in the examples, a heatmap representation showing an experimental comparison of lysates from normal kidney tissue (NK) and tumor kidney (RCC) tissue in the presence of a kinase inhibitor.

### DETAILED DESCRIPTION OF THE INVENTION

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

The present invention provides methods and devices that enable the diagnosis typing and/or subtyping of renal cell carcinoma based on the measurement of the kinase activity of a RCC tumor sample.

Preferably, in one embodiment of the present invention, methods are provided wherein the kinase activity is protein kinase activity. For purposes of the present invention, and as used herein the term "enzyme activity", "kinase activity" or "protein kinase activity" refer to the formation of reaction product(s) by a certain amount of enzyme, kinase or protein kinase acting on a substrate during the course of the assay.

Protein kinase activity is referred to as the activity of protein kinases. A protein kinase is a generic name for all enzymes that transfer a phosphate to a protein. About three to four percent of the human genome contains transcription information for the formation of protein kinases. Currently, there are about 518 known different protein kinases. However, because three to four percent of the human genome is a code for the formation of protein kinases, there may be many more separate kinases in the human body.

A protein kinase is a kinase enzyme that modifies other proteins by chemically adding phosphate groups to them. This process or activity is also referred to as phosphorylation. Phosphorylation can therefore be regarded as the process of the addition of a phosphate group to a substrate. Phosphorylation usually results in a functional change of the substrate by changing enzyme activity, cellular location, or association with other proteins. Up to 30% of all proteins may be modified by kinase activity, and kinases are known to regulate the majority of cellular pathways, especially those involved in signal transduction, the transmission of signals within the cell. The chemical activity of a kinase involves removing a phosphate group from ATP or GTP and covalently attaching it to amino acids such as serine, threonine, tyrosine, histidine aspartic acid and/or glutamic acid that have a free hydroxyl group. Most known kinases act on both serine and threonine, others act on tyrosine, and a number act on all serine, threonine and tyrosine. The protein kinase activity monitored with the method of the present invention is preferably directed to protein kinases acting towards serine, threonine and/or tyrosine, preferably acting on both serine and threonine, on tyrosine or on serine, threonine and tyrosine. According to the method of the present invention the protein kinase activity is directed to tyrosine protein kinases and more preferably the method of the present invention if preferably directed to protein kinases acting towards tyrosines.

Protein kinases are distinguished by their ability to phosphorylate substrates on discrete sequences. These sequences have been determined by sequencing the amino acids around the phosphorylation sites and are usually distinct for each protein kinase. The recognition sequence on each substrate is specific for each kinase catalyst.

Because protein kinases have profound effects on a cell, their activity is highly regulated. Kinases are turned on or off by for instance phosphorylation, by binding of activator proteins or inhibitor proteins, or small molecules, or by controlling their location in the cell relative to their substrates. Deregulated kinase activity is a frequent cause of disease, particularly cancer, where kinases regulate many aspects that control cell growth, movement and death. Therefore monitoring the protein kinase activity in tissues can be of great importance and a large amount of information can be obtained when comparing the kinase activity of different tissue samples.

As described in the present invention, the inventors have surprisingly found that the diagnosis of renal cell carcinoma can be determined on the basis of the measurement of the kinase activity of a kidney sample. As used in the present invention the term "diagnosis", "diagnose" of "diagnosing" refers to the process of identifying a medical condition or disease, and in the present case RCC, from the results of the methods according to the present invention. The conclusion reached through this diagnostical process is called a diagnosis. The measurement of the kinase activity is performed by contacting a kidney sample with one or more substrates, preferably protein kinase substrates, thereby generating a phosphorylation profile.

Said protein kinase substrates as used herein, are preferably peptides, proteins or peptide mimetics. The protein kinase substrates each comprise, preferably one or more, phosphorylation sites that can be phosphorylated by the protein kinases present in the sample. Therefore, exposure of a protein kinase substrate to a sample comprising a protein kinase results in the phosphorylation of one or more of the phosphorylation sites of the protein kinase substrate. This phosphorylation activity can be measured using techniques known in the art. Therefore, during the measurement method the kinase enzymes present in the sample will phosphorylate, preferably one or more, of the phosphorylation sites on one or more protein kinase substrates. The inventors have observed essential differences between kinase activity of tumors from RCC patients and the kinase activity of normal kidney tissue. Consequently, the inventors have observed that the kinases present in a RCC sample will phosphorylate protein kinase substrates differently compared to a normal kidney tissue sample. The effect has been observed to be even more significant when measurements in the absence of a protein kinase inhibitor are compared to measurements in the presence of a protein kinase inhibitor.

The present invention therefore provides a method for diagnosing renal cell carcinoma in a subject. In a first embodiment of the present invention, the method comprises the steps of:
(a) measuring kinase activity of a kidney lysate sample from said subject, by contacting said sample with at least one protein kinase substrate, thereby providing a phosphorylation profile of said kidney sample; and,
(b) determining from said phosphorylation profile the presence or absence of renal cell carcinoma in said subject, thereby diagnosing renal cell carcinoma in said subject.

As referred to in the present application Renal Cell Carcinoma (RCC) regards a specific type of kidney cancer which occurs very frequently. There are several known sub-types of RCC: clear cell subtype, chromophobic subtype, chromophilic subtype, papillary carcinoma subtype, oncocytic subtype and collecting duct (Bellini's duct) tumors.

As used in the present invention, the term "sample" refers to a sample obtained from an organism (patient) such as human or from components (e.g. tissue or cells) of such an organism. Said sample is preferably obtained from the kidney tissue of said patient and is referred to as "kidney sample". For the purpose of diagnosing said sample can either be a normal kidney sample, or a kidney tumor sample. For the purpose of typing, subtyping and predicting the response to a medicament said sample is preferably a kidney tumor sample derived from the tumor tissue of said patient. More preferably said kidney tumor sample is a RCC tumor tissue biopsy, vacuum assisted biopsy, fine needle biopsy, open surgical biopsy, vacuum assisted biopsy, or material from a resected tumor. Said kidney tumor sample is thereby referred to as a 'clinical sample' which is a kidney tumor sample derived from a RCC patient.

Said sample is preferably a fresh or a fresh frozen sample.

In particular, said sample refers to a cell lysate of a RCC tumor tissue obtained through tumor tissue biopsy, fine needle biopsy, open surgical biopsy or material from a resected tumor. Alternatively said sample may be obtained from specific RCC tumor cell lines and in particular cell lysates thereof.

Alternatively said sample may be derived from a tumor sample that has been cultured in vitro for a limited period of time.

In a preferred embodiment of the present invention said sample is a sample that has undergone a preparation step prior to the steps according to the method of the present invention. Preferably said preparation step is a step where the protein kinases present in said sample are released from the tissue by lysis. Additionally the kinases in the sample may be stabilized, maintained, enriched or isolated, and the measurement of the kinase activity as performed in step (a) occurs on the enriched or isolated protein kinase sample. By first enriching protein kinases in the sample or isolating protein kinases from the sample the subsequent measurement of the kinase activity will occur in a more efficient and reliable manner. Also the clarity and intensity of the obtained phosphorylation signal will be increased as certain contaminants are being removed during the enriching or isolating step.

As used in the present invention, the term "phosphorylation profile" refers to a data set representative for the phosphorylation levels of, preferably one or more, phosphorylation sites present on each protein kinase substrate. When measuring the kinase activity of a sample by contacting said sample with protein kinase substrates a specific phosphorylation profile is obtained. The phosphorylation profile is generated by the phosphorylation of the protein kinase substrates with the protein kinases present in the sample and it comprises the level of phosphorylation of the phosphorylation sites present on the protein kinase substrates used. A phosphorylation profile can thus be generated when using at least one protein kinase substrate in different test conditions such as for example by comparing the phosphorylation level of a sample on one peptide or protein (protein kinase substrate) in the presence and absence of a protein kinase inhibitor or by comparing the phosphorylation level of a sample on one peptide or protein (protein kinase substrate) with a known phosphorylation level indicative for a non-tumorous tissue. More frequently phosphorylation profiles of a sample will be measured using several protein kinase substrates in the same or sequentially carried out experiments. Preferably, the present invention determines tyrosine kinase activity levels or profiles.

It should be noted that a person skilled in the art will appreciate that the methods of the present invention can use phosphorylation profiles as a basis for diagnosing, typing and/or subtyping RCC or as a basis for predicting the response of the tumor to a medicament. However, the phosphorylation levels of individual protein kinase substrates can also be used as a basis for diagnosing, typing and/or subtyping RCC or as a basis for predicting the response of the tumor to a medicament.

It should be noted that for the measurement of the protein kinase activity, ATP or any other phosphate source needs to be added to the sample when it is contacted with the protein kinase substrates. The presence of ATP will lead to a phosphorylation of the protein kinase substrates. Alternatively, the phosphorylation of the protein kinase substrates can be performed in the absence of exogenous ATP. When no ATP is added during the incubation of the sample with the protein kinase substrates, the endogenous ATP, the ATP naturally present in the sample, will act as the primary source of ATP.

The phosphorylation level of each of the protein kinase substrates can be monitored using any method known in the art. The response of the protein kinase substrates is determined using a detectable signal, said signal resulting from the interaction of the sample with the protein kinase substrates or by for instance measuring mass differences using mass spectrometry. In determining the interaction of the sample with the protein kinase substrates the signal is the result of the interaction of the phosphorylated substrates with a molecule capable of binding to the phosphorylated substrates. This binding can be detected by e.g. surface plasmon resonance or by the molecule being detectably labelled. For the latter, the molecule that specifically binds to the substrates of interest (e.g. antibody or polynucleotide probe) can be detectably labelled by virtue of containing an atom (e.g. radionuclide), molecule (e.g. fluorescein), or enzyme or particle or complex that, due to a physical or chemical property, indicates the presence of the molecule. A molecule may also be detectably labelled when it is covalently bound to or otherwise associated with a "reporter" molecule (e.g. a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labelled avidin or streptavidin conjugate, magnetic beads (e.g. Dynabeads'), fluorescent dyes (e.g. fluorescein, fluorescein-isothiocyanate (FITC), Texas red, rhodamine, green fluorescent protein, enhanced green fluorescent protein, lissamine, phycoerythrin, Cy2, Cy3, Cy3.5, Cy5, Cy5.5, Cy7, FluorX [Amersham], SYBR Green I & II [Molecular Probes], and the like), radiolabels (e.g. 3H, 125I, 35S, 14C, or 32P), enzymes (e.g. hydrolases, particularly phosphatases such as alkaline phosphatase, esterases and glycosidases, or oxidoreductases, particularly peroxidases such as horse radish peroxidase, and the like), substrates, cofactors, chemilluminescent groups, chromogenic agents, and colorimetric labels such as colloidal gold or coloured glass or plastic (e. g. polystyrene, polypropylene, latex, etc.), protein particles or beads.

Means of detecting such labels are well known to those of skill in the art. Thus, for example, chemiluminescent and radioactive labels may be detected using photographic film or scintillation counters, and fluorescent markers may be detected using a photodetector to detect emitted light (e.g. as in fluorescence-activated cell sorting). Enzymatic labels are typically detected by providing the enzyme with a substrate and detecting a coloured reaction product produced by the action of the enzyme on the substrate. Colorimetric labels are detected by simply visualizing the coloured label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the colour associated with the label. Fluorescence resonance energy transfer has been adapted to detect binding of unlabeled ligands, which may be useful on arrays.

In a particular embodiment of the present invention the response of the protein kinase substrates to the sample is determined using detectably labelled antibodies; more in particular fluorescently labelled antibodies. In those embodiments of the invention where the substrates consist of protein kinase substrates, the response of the protein kinase substrates is determined using fluorescently labelled anti-phosphotyrosine antibodies, fluorescently labelled anti-phosphoserine or fluorescently labelled anti-phosphothreonine antibodies. The use of fluorescently labelled anti-phosphotyrosine antibodies or fluorescently labelled anti-phosphoserine or fluorescently labelled anti-phosphothreonine antibodies in the method of the present invention, allows real-time or semi real-time determination of the protein kinase activity and accordingly provides the possibility to express the protein kinase activity as the initial velocity of protein kinase derived from the activity over a certain period of incubation of the sample on the protein kinase substrates.

The inventors have found that measuring kinase activity of a kidney sample, enables diagnosis of patients suffering from RCC. It has been found by monitoring kinase activity in normal kidney tissue and kidney tumor tissue, that there is a prominent difference between the kinase activity of tumourous and normal kidney tissue. This difference enables the accurate diagnosis as well as the typing and/or subtyping of RCC as well as the response prediction of RCC to a medicament. Furthermore, a set of peptide markers have shown to provide a good way for the diagnosis, typing, subtyping and/or response prediction of RCC according to the methods of the present invention. The use of a set of peptide markers provides more accurate methods using and moreover a limited number of protein kinase substrates.

It should further be noted that phosphorylation profiles and levels can also be determined according to the method of the present invention wherein the phosphorylation profiles or levels of a kidney tumor sample are compared to a normal kidney sample. Preferably both samples are obtained from the same patient.

In another embodiment according to the present invention, the phosphorylation profile comprises the phosphorylation levels of, preferably one or more, phosphorylation site(s) present in any of the peptide markers as listed in Table 1 and preferably SEQ ID NO 1 to 44. Preferably phosphorylation levels will be studied of, preferably one or more, phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46 of the peptide markers as listed in Table 1 and preferably phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 or 44 of the peptide markers with SEQ ID NO 1 to 44.

The term "peptide markers" in the context of the present invention refers to the fact that the peptides as listed in Table 1 can be preferably used according to the methods of the present invention as target regions to measure the phosphorylation levels of phosphorylation sites of said markers in the presence of protein kinase present in samples. The phosphorylation levels of the individual phosphorylation sites present in said markers may be measured and compared in different ways. Therefore the present invention is not limited to the use of peptides identical to any of these peptide markers as listed in Table 1 as such. The skilled person may easily on the basis of the peptide markers listed in Table 1 design variant peptides compared to the specific peptides in said Table and use such variant peptides in a method for measuring phosphorylation levels of phosphorylation sites common to said peptide markers as listed in Table 1. These variant peptides may have one or more (2, 3, 4, 5, 6, 7, etc.) amino acids more or less than the given peptides and may also have amino acid substitutions (preferably conservative amino acid substitutions) as long as these variant peptides retain at least one or more of the phosphorylation sites of said original peptides as listed in said table. Further the skilled person may also easily carry out the methods according to the present invention by using proteins (full length or N- or C-terminally truncated) comprising the amino acid regions of the "peptide markers" listed in Table 1 as sources for studying the phosphorylation of sites present in the amino acid regions of the peptides listed in Table 1. Also the skilled person may used peptide mimetics.

The protein kinase substrates as used in the methods described herein are meant to include peptides, proteins or peptide mimetics comprising, preferably one or more, of the phosphorylation sites of the peptide markers of Table 1. Said one or more phosphorylation sites are specifically phosphorylated by the protein kinases present in the sample thereby providing a phosphorylation profile. More preferably the protein kinase substrates (peptides, proteins or peptide mimetics) as used in the method of the present invention comprise, preferably one or more, of the phosphorylation sites present in at least two peptide markers as listed in Table 1 and preferably SEQ ID NO 1 to 44. More particularly said protein kinase substrates represent the, preferably one or more, phosphorylation sites present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46 of the peptide markers as listed in Table 1 and preferably phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 or 44 of the peptide markers with SEQ ID NO 1 to 44. In a more preferred embodiment the protein kinase substrates comprise or consist of, preferably one or more, phosphorylation sites present in all of the peptide markers listed in Table 1 and preferably SEQ ID NO 1 to 44.

A person skilled in the art will appreciate that the phosphorylation sites present in a single peptide marker as listed in Table 1 enable determining the diagnosis of RCC. The peptide marker as listed in Table 1 also enable the typing, subtyping and the medicament response prediction of RCC. However, when the number of peptide markers as listed in Table 1 increases, so will increase the specificity and sensitivity of the method according to the present invention. When for example only one protein kinase substrate comprising the phosphorylation sites of a single peptide marker as listed in table 1 is used for the diagnosis of RCC the accuracy of the method will be lower, compared to a method where the diagnosis of RCC uses multiple protein kinase substrates comprising the phosphorylation sites of multiple peptide markers as listed in table 1. The highest method accuracy will be obtained when all protein kinase substrates comprising the phosphorylation sites of all peptide markers as listed in table 1 are used.

Table 1: list of 46 peptide markers comprising phosphorylation sites used for determining the kinase activity, their sequence and SEQ ID NO The name of the peptide markers refers to the associated proteins and also refers to the start and the end position of the amino acid sequence.

| **SEQ ID NO** | **Peptide marker Name** | **Peptide marker Sequence** |
|---|---|---|
| 1 | TNNT1_2_14 | SDTEEQEYEEEQP |
| 2 | EPHA2_765_777 | EDDPEATYTTSGG |
| 3 | JAK2_563_577 | VRREVGDYGQLHETE |
| 4 | PAXI_111_123 | VGEEEHVYSFPNK |
| 5 | FER_707_719 | RQEDGGVYSSSGL |
| 6 | EPHB1_771_783 | DDTSDPTYTSSLG |
| 7 | P85A_600_612 | NENTEDQYSLVED |
| 8 | FGFR2_762_774 | TLTTNEEYLDLSQ |
| 9 | CBL_693_705 | EGEEDTEYMTPSS |
| 10 | PGFRB_1014_1028 | PNEGDNDYIIPLPDP |
| 11 | LAT_194_206 | MESIDDYVNVPES |
| 12 | EPHA7_607_619 | TYIDPETYEDPNR |
| 13 | RON_1346_1358 | SALLGDHYVQLPA |
| 14 | LAT_249_261 | EEGAPDYENLQEL |
| 15 | SRC8_CHICK_492-504 | YQAEENTYDEYEN |
| 16 | EPOR_361_373 | SEHAQDTYLVLDK |
| 17 | CD79A_181_193 | EYEDENLYEGLNL |
| 18 | FRK_380_392 | KVDNEDIYESRHE |
| 19 | VGFR1_1235_1247 | ATSMFDDYQGDSS |
| 20 | FAK2_572_584 | RYIEDEDYYKASV |
| 21 | PGFRB_572_584 | VSSDGHEYIYVDP |
| 22 | DYR1A_312_324 | CQLGQRIYQYIQS |
| 23 | PLCG1_764_776 | IGTAEPDYGALYE |
| 24 | FES_706_718 | REEADGVYAASGG |
| 25 | PDPK1_2_14 | ARTTSQLYDAVPI |
| 26 | ANXA1_14_26 | IENEEQEYVQTVK |
| 27 | PECA1_706_718 | KKDTETVYSEVRK |
| 28 | SRC8_CHICK_476_488 | EYEPETVYEVAGA |
| 29 | ENOG_37_49 | SGASTGIYEALEL |
| 30 | EPHA1_774_786 | LDDFDGTYETQGG |
| 31 | RET_1022_1034 | TPSDSLIYDDGLS |
| 32 | PAXI_24_36 | FLSEETPYSYPTG |
| 33 | PDPK1_369_381 | DEDCYGNYDNLLS |
| 34 | K2C6B_53_65 | GAGFGSRSLYGLG |
| 35 | VGFR1_1326_1338 | DYNSVVLYSTPPI |
| 36 | ERBB2_870_882 | LDIDETEYHADGG |
| 37 | EGFR_1165_1177 | ISLDNPDYQQDFF |
| 38 | JAK1_1015_1027 | AIETDKEYYTVKD |
| 39 | MBP_198_210 | ARTAHYGSLPQKS |
| 40 | PGFRB_709_721 | RPPSAELYSNALP |
| 41 | VGFR2_989_1001 | EEAPEDLYKDFLT |
| 42 | VGFR2_1052_1064 | DIYKDPDYVRKGD |
| 43 | RASA1_453_465 | TVDGKEIYNTIRR |
| 44 | Abl_artificial_kinase_substrate | EAIYAAPFAKKK |
| 45 | CTNB1_79_91 | VADIDGQYAMTRA |
| 46 | PGFRB_771_783 | YMAPYDNYVPSAP |

It should further be noted that according to a preferred embodiment of the present invention the peptide markers as listed in Table 1 can be used as such for carrying out the methods according to the present invention. The present invention however also includes the use of analogs and combinations of these peptide markers for use in the method according to the present invention. The peptide marker analogs include peptide markers which show a sequence identity of more than 70%, preferably more than 80% and more preferably more than 90%.

In yet another embodiment, the present invention relates to a method according to the present invention wherein said kinase activity of said kidney sample from said subject is measured in the presence and in the absence of a protein kinase inhibitor, thereby providing a phosphorylation profile of said sample in the presence and in the absence of a protein kinase inhibitor; and, determining from said phosphorylation profiles in the presence and in the absence of a protein kinase inhibitor the differential phosphorylation level, said differential phosphorylation level indicating the presence or absence of renal cell carcinoma in said subject.

The inventors have further found that by comparing phosphorylation profiles in the presence and in the absence of a protein kinase inhibitor the difference between experimental variation can be reduced. Moreover, the measurement of the kinase activity of said sample preferably occurs by contacting said sample with at least one protein kinase substrate in the presence and in the absence of a protein kinase inhibitor. Techniques from the prior art often require the incubation of the cells or tissues with said compounds preferably in vivo, during the culturing of the cells or tissues or during a large time period prior to the actual measurement of the kinase activity. The present invention provides that the protein kinase inhibitor is added directly to the sample and preferably directly to the lysate sample. The protein kinase inhibitors are added to the sample only just prior to contacting the sample with the protein kinase substrates and performing the kinase activity assay. Consequently, the protein kinase inhibitors are added in vitro at the time the incubation of the lysate sample with the protein kinase substrates is initiated. The present invention therefore provides an in vitro primary screening tool which allows the use of a single sample which is split into a first part that is used for the incubation of the sample in the absence of a protein kinase inhibitor while a second part of the sample is used for the incubation of the sample in the presence of a protein kinase inhibitor.

The term "differential phosphorylation level" as used herein therefore refers to a data set comprising comparison data from the phosphorylation profiles in the presence and in the absence of a protein kinase inhibitor. The statistical analysis of the differential phosphorylation level can be done using multivariate and/or univariate statistical methods known in the art. The differential phosphorylation levels are obtained by (numerically) comparing the peptide phosphorylation levels or profiles in the presence and in the absence of the protein kinase inhibitor in the same sample, for instance, but not limited to, providing ratios or differences of the profiles obtained in the presence and the absence of the protein kinase inhibitor.

In addition, because the differential phosphorylation level is generated by comparing the phosphorylation levels or profiles of the same sample in the presence and the absence of the protein kinase inhibitor, preferably during a parallel series of measurements run in the same instrument, the differential phosphorylation level is surprisingly found to be less affected by variation, for example biological variation, experimental variation, compared to single phosphorylation levels or profiles. This provides a more robust, more sensitive, more reproducible and more reliable method for diagnosing, typing, subtyping and/or response prediction of RCC.

According to another embodiment, the present invention relates to a method according to the present invention wherein additionally a classifier parameter is established from said phosphorylation profile(s) of said sample, said classifier parameter determining the presence or absence of renal cell carcinoma in said subject.

By establishing a classifier parameter for diagnosing, typing and/or subtyping RCC the method of the present invention a criterion is established for analysing the results obtained from the method of the present invention. This criterion enables a person to provide a diagnosis on the basis of a single or limited number of data. The person providing the diagnosis does not have to interpret an entire set of data, but rather bases his conclusion on the basis of a single or limited number of criteria.

The term "classifier parameter" as used herein represents a discriminating value which has been determined by establishing a single phosphorylation profile or a phosphorylation profile in the presence and in the absence of a protein kinase inhibitor. Said discriminating value identifies the diagnosis of RCC. Since the classifier parameter includes information regarding the phosphorylation level of several protein kinase substrates. Classification is a procedure in which individual items are placed into groups based on quantitative information on one or more characteristics inherent in the items (e.g. phosphorylation levels or profiles of a sample) and based on a training set of previously labelled items (clinical response to a pharmacotherapy). A classifying parameter is calculated by applying a "classifier" to the measured phosphorylation levels of a sample. Based on the classifying parameter a sample is assigned to (or predicted to belong to) a class RCC or normal kidney tissue or in another embodiment of the present invention to instance RCC patient tissue responsive or non-responsive to one or more protein kinase inhibitors. The classifier has been previously determined by comparing samples which are known to belong to the respective relevant classes. For instance the classifier may be a mathematical function that uses information regarding the phosphorylation level of several protein kinase substrates which individual protein kinase substrates can be weighted based on the measured phosphorylation level of a number of protein kinase substrates (or values derived from that). Several methods are known in the art for developing a classifier including the neural network (Multi-layer Perceptron), support vector machines, k-nearest neighbours, Gaussian mixture model, naive bayes, decision tree, RBF classifiers, random forest, disciminant analysis, linear discriminant analysis, quadratic discriminant analysis, discriminant analysis - principal component analysis, partial least squares discriminant analysis, generalized distance regression and elastic net classification.

It is not relevant to give an exact threshold value for the classifier parameter. A relevant threshold value can be obtained by correlating the sensitivity and specificity and the sensitivity/specificity for any threshold value. A threshold value resulting in a high sensitivity results in a lower specificity and vice versa. If one wants to increase the positive diagnostical value of the test to diagnose RCC with a high certainty, then the threshold value of the test can be changed which as a consequence will decrease the negative diagnostical value of the test to diagnose RCC negative patients. If one wants to increase the negative diagnostical value of the test to diagnose RCC then the threshold value can be changed in the opposite direction which as a consequence will decrease the positive diagnostical value of the test to diagnose RCC

It is thus up to the individual diagnostic engineers to determine which level of positive predictive value/negative predictive value/sensitivity/specificity is desirable and how much loss in positive or negative predictive value is tolerable. The chosen threshold level could be dependent on other diagnostic parameters used in combination with the present method developed by the diagnostic engineers.

In yet another embodiment, the present invention relates to a method according to the present invention wherein said classifier parameter indicates the presence of renal cell carcinoma in said subject if said classifier parameter is above a first predetermined threshold level, and wherein said classifier parameter indicates the absence of renal cell carcinoma in said subject if said classifier parameter is below a second predetermined threshold level.

According to another embodiment, the present invention relates to the method of the present invention wherein said phosphorylation profile, differential phosphorylation profile or level or said classifier parameter indicates the presence or absence of renal cell carcinoma in said subject or provides an undetermined or intermediate diagnosis.

As used in the present application the diagnosis of RCC is generally divided into two types of diagnosis, either the presence or absence of renal cell carcinoma in said subject and additionally some diagnoses may be undetermined or intermediate.

In another embodiment, the present invention regards the method according to the present invention wherein said peptide markers are at least two peptide markers selected from the group consisting of the peptide markers with any of SEQ ID NO 1 to 44.More preferably the present invention relates to a method according to the present invention wherein said protein kinase inhibitor is sorafenib or sunitinib.

As used herein, the term "protein kinase inhibitor" refers to a type of enzyme inhibitor which blocks the action of one or more protein kinases, hence they can be subdivided or characterised by peptides or proteins whose phosphorylation is inhibited. Examples of protein kinase inhibitors for use in the method of the present invention are Dasatinib (currently used for the treatment of leukaemia); erlotinib (currently used for the treatment of non-small cell lung cancer); gefitinib (currently used for the treatment of non-small cell lung cancer); imatinib (currently used for the treatment of gastrointestinal stromal tumors and leukaemia); lapatinib (currently used for the treatment of breast cancer); nilotinib (currently used for the treatment of leukaemia); sorafinib (currently used for the treatment of renal cell carcinoma and hepatocellular carcinoma; Sunitinib (currently used for the treatment of renal cell carcinoma); temsirolimus (currently used for the treatment of renal cell carcinoma); ABT-869; AEE788; Alvocidib; AP23464; AP23846; AP23848; ARRY-142886; ARRY-334543; AT-7519; Axitinib; AZD0530; AZD1152; BIBW-2992; BIRB-796; BMI-1026; BMS-599626; Bosutinib; Brivanib; Canertinib; CCT129202; Cediranib; CEP-7055; CP-547632; CP-724714; Dovitinib; E7080; Enzastaurin; everolimus; FI-700; Gossypol; HKI-272; HMN-176; HMN-214; INNO-406; JNJ-7706621; KRX-0601; LBW242; Lestaurtinib; Midostaurin; MK-0457; MLN8054; MP-470; Neratinib; ON0123380; ON01910; ON-01910; OSI-930; Pazopanib; PD166326; PD173955; PD180970; Pelitinib; PF-2341066; PHA665752; PHA-739358; PX-866; R-547; Seliciclib; Semapimod; Semaxanib; SNS-032; SU011248; SU014813; SU11248; SU11274; SU14813; Tandutinib; Telatinib; TSU-68; UCN-01; Vandetanib; Vatalanib; VE-465; ZM 447439 and protein kinase inhibitors used in research including Tyrphostin-1; Tyrphostin-23; Tyrphostin-51; Tyrphostin-63; AG-1007; AG-1112; AG-1433; RG-13022; SU-1498; I-OMe-Tyrphostin; AG-538; Protein Kinase G inhibitor peptide (Arg-Lys-Arg-Ala-Arg-Lys-Glu); Geldanamycin from Streptomyces hygroscopicus; Lavendustin A; and Genistein. More preferably for the purpose of the present invention, said protein kinase inhibitors are protein kinase inhibitors chosen from the group comprising sorafenib, Pazopanib and/or sunitinib.

Additionally, the inventors have further found that by adding additional protein kinase inhibitors during the measurement of the kinase activity the method of the present invention allows further differentiation between the obtained phosphorylation profiles. When using both a first and a second protein kinase inhibitor while measuring the kinase activity, four different phosphorylation profiles can be obtained: a phosphorylation profile in the absence of any protein kinase inhibitors, a phosphorylation profile in the presence of the first protein kinase inhibitor, a phosphorylation profile in the presence of the second protein kinase inhibitor and a phosphorylation profile in the presence of the first and the second protein kinase inhibitor. A person skilled in the art can perform this with "n" number of protein kinase inhibitors, "n" being an integer of one or more.

Another embodiment of the present invention relates to a method according to the present invention wherein said kinase substrates carrying phosphorylation sites are located or immobilized on a solid support, and preferably a porous solid support. Preferably said immobilized kinase substrates carrying phosphorylation sites will be immobilized proteins, peptides or peptide mimetics. In a preferred embodiment of the present invention peptides are immobilized on a solid support.

As used herein "peptide" refers to a short truncated protein generally consisting of 2 to 100, preferably 2 to 30, more preferably 5 to 30 and even more preferably 13 to 18 naturally occurring or synthetic amino acids which can also be further modified including covalently linking the peptide bonds of the alpha carboxyl group of a first amino acid and the alpha amino group of a second amino acid by eliminating a molecule of water. The amino acids can be either those naturally occurring amino acids or chemically synthesized variants of such amino acids or modified forms of these amino acids which can be altered from their basic chemical structure by addition of other chemical groups which can be found to be covalently attached to them in naturally occurring compounds.

As used herein "protein" refers to a polypeptide made of amino acids arranged in a linear chain and joined together by peptide bonds between the carboxyl and amino groups of adjacent amino acid residues.

As used herein "peptide mimetics" refers to organic compounds which are structurally similar to peptides and similar to the peptide sequences list in Table 1. The peptide mimetics are typically designed from existing peptides to alter the molecules characteristics. Improved characteristics can involve, for example improved stability such as resistance to enzymatic degradation, or enhanced biological activity, improved affinity by restricted preferred conformations and ease of synthesis. Structural modifications in the peptidomimetic in comparison to a peptide, can involve backbone modifications as well as side chain modification.

For measuring the kinase activity of the sample a large variety of methods and formats are known in the art. The kinase activity can for example be measured using ELISA and multiplex ELISA techniques, blotting methods, mass spectrometry, capillary electrophoresis, bead arrays, macroarrays, microarrays or any other method known in the art. Depending on the type of kinase activity measurement method the solid support on which the proteins, peptides or peptide mimetics are fixed may vary. Whereas in ELISA the protein kinase substrates are attached to the surface of the microtiterplates, in microarrays the protein kinase substrates are immobilized on and/or in the microarray substrate.

In a preferred embodiment of the present invention the protein kinase substrates are immobilized on an array, and preferably a microarray of protein kinase substrates wherein the protein kinase substrates are immobilized onto a solid support or another carrier. The immobilization can be either the attachment or adherence of two or more protein kinase substrate molecules to the surface of the carrier including attachment or adherence to the inner surface of said carrier in the case of e.g. a porous or flow-through solid support.

It is also disclosed an array of protein kinase substrates being a flow-through array. The flow-through array as used herein could be made of any carrier material having oriented through-going channels as are generally known in the art, such as for example described in PCT patent publication WO 01/19517. Typically the carrier is made from a metal oxide, glass, silicon oxide or cellulose. In a particular embodiment the carrier material is made of a metal oxide selected from the group consisting of zinc oxide, zirconium oxide, tin oxide, aluminium oxide, titanium oxide and thallium, in a more particular embodiment the metal oxide consists of aluminium oxide.

Accordingly, in a further embodiment of the methods of the present invention said array is a Pamchip®.

In a further embodiment, the present invention relates to a method according to the present invention wherein said solid support (microarray) comprises any of the peptides as listed in Table 1 immobilized thereto.

In a further embodiment, the present invention relates to a method according to the present invention wherein said solid support (microarray) comprises each of the peptide as listed in Table 1 immobilized thereto.

Another embodiment of the present invention regards a method for predicting the response of a patient diagnosed with renal cell carcinoma to a medicament, wherein the kinase activity of a sample, obtained from the renal cell carcinoma from said patient, is measured in the presence and in the absence of said medicament and wherein said kinase activity in the presence said medicament is compared to the kinase activity in the absence of said medicament thereby determining the response of said patient to said medicament, wherein said kinase activity measurement provides phosphorylation profiles of said sample in the presence and in the absence of said medicament.

It should be noted that the observed response of the patient to said medicament can either be a positive response, wherein the medicament will improve the treatment of said patient, or a negative response, wherein the medicament has a negative or no influence on the treatment of said patient.

By measuring the kinase activity of a sample, obtained from the RCC tumor from said patient, in the presence and in the absence of a medicament, the effect of that medicament to the RCC can be assessed. This method was found particularly useful in the prediction of response to said medicament, and to enable the distinction between responders and non-responders in the treatment with said medicament. The measurement of the kinase activity of said sample preferably occurs by contacting said sample with at least one protein kinase substrate in the presence and in the absence of said medicament. Techniques from the prior art often require the incubation of the cells or tissues with said medicaments preferably in vivo, during the culturing of the cells or tissues or during a large time period prior to the actual measurement of the kinase activity. The present invention provides that the medicament is added directly to the sample and preferably directly to the lysate sample. The medicament is added to the sample only just prior to contacting the sample with the protein kinase substrates and performing the kinase activity assay. Consequently, the medicament is added in vitro at the time the incubation of the lysate sample with the protein kinase substrates is initiated. The present invention therefore provides an in vitro primary screening tool which allows the use of a single sample which is split into a first part that is used for the incubation of the sample in the absence of a medicament while a second part of the sample is used for the incubation of the sample in the presence of a medicament.

The medicament as used in the method of the present invention can be any kind of chemical substance for instance used in the treatment, cure, prevention, or diagnosis of disease or used to otherwise enhance physical or mental well-being. Specifically said medicament can be a kinase inhibitor, and more preferably a protein kinase inhibitor, and more preferably a sorafenib and/or sunitinib protein kinase inhibitor and most preferably a small molecule protein kinase inhibitor.

Such a response prediction is of great value as it enables the selection of patients for highly beneficial treatment with drugs such as sorafinib or sunitinib. It prevents the overtreatment of RCC patients with drugs such as sorafinib or sunitinib, especially since a majority of the patients do not actually show a clinical response. Response prediction further reduces the expenses on highly priced sunitinib and sorafinib in non-responsive patient treatments and the improvement of the experiences on patient selection are expected to lead the way to personalized treatments, thereby improving other targeted drug developments.

In another embodiment of the present invention the method for predicting the response of a patient diagnosed with renal cell carcinoma to a medicament, uses phosphorylation profiles which comprise the phosphorylation levels of, preferably one or more, phosphorylation sites present in any of the peptide markers as listed in table 1 and preferably SEQ ID NO 1 to 44. Preferably also this method will use two or more of said peptide markers as described above. More preferably this method uses, preferably one or more, phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46 of the peptide markers as listed in Table 1 and preferably phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 or 44 of the peptide markers with SEQ ID NO 1 to 44.

Also for this embodiment, the amount and the type of peptides, proteins or peptide mimetics to be used is as described above. Phosphorylation levels can also be measured according to the invention, without the necessity to generate phosphorylation profiles thereof.

It is clear that effects of a medicament can be monitored using this method. The medicament affects the degree of inhibition, the potency and/or the selectivity of the kinases in the sample. More peptide inhibition is caused by the larger effect of the medicament on the kinases in the sample and therefore the drug is less effective. Also an increased peptide inhibition would lead to a larger amount of normal tissues being affected by the drug, making the drug less tumor tissue specific.

An array for carrying out the methods of the present invention is also disclosed, said array comprising immobilized proteins, peptides or peptide mimetics comprising, preferably one or more, phosphorylation sites present in any of the peptide markers as listed in table 1 and preferably SEQ ID NO 1 to 44. More preferably said array comprises immobilized proteins, peptides or peptide mimetics comprising, preferably one or more, phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46 of the peptide markers as listed in Table 1 and preferably phosphorylation site(s) present in at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 or 44 of the peptide markers with SEQ ID NO 1 to 44.

Said array comprises immobilized proteins, peptides or peptide mimetics comprising, preferably one or more, phosphorylation sites present in at least 1, 2, 3, 4, 5, 6, 7, 8 or 9 of the peptide markers selected from the group consisting of the peptide markers with any of SEQ ID NO 40, 25, 12, 30, 6, 2, 33, 45 and/or 13.

Said array comprises immobilized proteins, peptides or peptide mimetics comprising, preferably one or more, phosphorylation sites present in at least 1, 2, 3, 4, 5, 6, 7, 8, 9, 10, 11, 12, 13 or 14 of the peptide markers selected from the group consisting of the peptide markers with any of SEQ ID NO 46, 29, 3, 7, 15, 21, 27, 18, 4, 28, 24, 5, 16 and/or 17.

Said array comprises immobilized proteins, peptides or peptide mimetics comprising, preferably one or more, phosphorylation sites present in at least 1, 2, 3, 4, 5, 6, 7, 8, 9, 10, 11, 12, 13 or 14 of the peptide markers selected from the group consisting of the peptide markers with any of SEQ ID NO 35, 32, 36, 34, 26, 20, 19, 10, 11, 14, 1, 9, 8 and/or 38.

Said proteins, peptides or peptide mimetics are preferably at least 25% of proteins, peptides or peptide mimetics on said array.

More particularly said array comprises immobilized proteins, peptides or peptide mimetics comprising, preferably one or more, phosphorylation sites as described in detail above representing the peptide markers as listed in table 1. Additionally said proteins, peptides or peptide mimetics are preferably at least 25%, at least 50%, at least 70%, at least 80%, at least 90% or 100% of the proteins, peptides or peptide mimetics on said array.

The type of arrays to be used are known in the art and are further detailed above. Said arrays may further comprise one or more immobilized proteins, peptides or peptide mimetics which are used as calibration means for performing the methods according to the present invention.

The present invention also relates in another embodiment to a computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out a method according to the present invention.

The present invention further relates to a computer system comprising a processor, and a memory coupled to said processor and encoding one or more programs, wherein said one or more programs instruct the processor to carry out a method according to the present invention.

The present application also provides a kit for diagnosing, typing or subtyping renal cell carcinoma, comprising at least one array according to the present invention, and optionally a computer readable medium having recorded thereon one or more programs for carrying out the method according to the present invention.

The present invention further relates in yet another embodiment to a method for typing or subtyping renal cell carcinoma, comprising the steps of:
(a) measuring the kinase activity of a renal cell carcinoma sample according to the method of the present invention, thereby providing a phosphorylation profile of said renal cell carcinoma sample; and,
(b) determining from said phosphorylation profile the type or subtype of renal cell carcinoma.

The method of the present invention enables the typing and/or subtyping of RCC into the RCC types and/or subtypes known in the art and types and/or subtypes as determined on the basis of the phosphorylation profiles. Subtypes of RCC include, but are not limited to, clear cell subtype, chromophobic subtype, chromophilic subtype, papillary carcinoma subtype, oncocytic subtype or collecting duct (Bellini's duct) tumors.

In a further embodiment of the present invention the invention regards a method for diagnosing and/or typing and/or subtyping renal cell carcinoma in a subject, comprising the steps of:
(a) measuring the kinase activity of a kidney sample from said subject, optionally in the presence and in the absence of a protein kinase inhibitor, thereby providing the phosphorylation level of, preferably one or more, phosphorylation sites present in any of the peptide markers as listed in Table 1; and,
(b) determining from said phosphorylation level, optionally in the presence and in the absence of a protein kinase inhibitor) the presence or absence of renal cell carcinoma in said subject.

Preferably, the present invention regards a method for diagnosing and/or typing and/or subtyping renal cell carcinoma in a subject according to any of the methods of the present invention wherein kinase activity of a kidney sample from said subject is measured thereby providing phosphorylation profile(s) or phosphorylation level(s) of, preferably one or more, phosphorylation sites present in any of the peptide markers as listed in Table 2a, 2b and/or 2c.

In another embodiment at least 1, 2, 3, 4, 5, 6, 7, 8 or 9 of the peptide markers selected from the group consisting of the peptide markers with any of SEQ ID NO 40, 25, 12, 30, 6, 2, 33, 45 and/or 13 are used in the methods of the present invention, more preferably for (sub)typing RCC.

In another embodiment at least 1, 2, 3, 4, 5, 6, 7, 8, 9, 10, 11, 12, 13 or 14 of the peptide markers selected from the group consisting of the peptide markers with any of SEQ ID NO 46, 29, 3, 7, 15, 21, 27, 18, 4, 28, 24, 5, 16 and/or 17 are used in the methods of the present invention, more preferably for (sub)typing RCC.

In another embodiment at least 1, 2, 3, 4, 5, 6, 7, 8, 9, 10, 11, 12, 13 or 14 of the peptide markers selected from the group consisting of the peptide markers with any of SEQ ID NO 35, 32, 36, 34, 26, 20, 19, 10, 11, 14, 1, 9, 8 and/or 38 are used in the methods of the present invention, more preferably for (sub)typing RCC.

Since the present inventors have identified a surprisingly useful set of peptide markers to be used in methods for diagnosing, typing or subtyping renal cell carcinoma, the skilled man may carry out any method as defined above wherein he measures the kinase activity of any of the peptide markers of Table 1. Also this method may be carried out using the amount and type of peptides, proteins or protein mimetics as defined above. The formats for carrying out this methods are also as for the methods described above.

The present invention is hereafter exemplified by the illustration of particular, non-limiting examples.

### EXAMPLES

### EXAMPLE 1 - Example determining the diagnostic set of 44 peptide markers

The present example demonstrates how the diagnostic set of peptide markers was established. Both normal and tumor kidney tissue were derived from resection material immediately after surgery. These tissues were snap-frozen and were stored at -85°C. The tissues were cut in slices of 10 µm and stored. For protein extraction 6 slices were lysed in 100 µL of Mammalian Protein Extraction Buffer (M-PER), a lysis buffer containing HALT protease and phosphatase inhibitors, for 60 minutes on ice. The protein concentration was determined for each sample and for the analysis on a peptide microarray comprising 144 peptides typically 5 µg of protein was used. For this analysis 5 µg of the proteins in the lysate was added to a kinase buffer (1x Abl kinase buffer from New England Biolabs), 100 µM of ATP, and 20 µg/mL of the fluorescently labelled antiphosphotyrosine antibody PY20 to an end volume of 40 µL. Before incubation of the lysate reaction mixtures on a PamChip® substrate array, a blocking step was carried out on the substrate arrays with 2% bovine serum albumin. After loading of the lysate reaction mixtures into substrate arrays comprising 144 protein kinase substrates, comprising the protein kinase peptide substrates as listed in Table 1, incubation was commenced thereby measuring the kinase activity of the sample.

To assess the effect of a kinase inhibitor, the kinase inhibitors sorafinib or sunitinib were added to the lysate before application onto the peptide microarray. During 60 cycles of pumping the lysate reaction mixture through the array, peptide phosphorylation was detected by the fluorescently labelled PY20 anti-phosphotyrosine antibody present in the lysate reaction mixture. Real time data were obtained by measuring fluorescence of the bound anti-phosphotyrosine antibody after each 5 cycles. Images of the array were taken during the incubation of the array and after 60 cycles of incubation. After 60 cycles of incubation and imaging, the antibody mixture was removed and the array was washed. Images were collected at different exposure times. Signals for each spot on the image were quantified. Image quantification and data processing was conducted with dedicated PamGene software (Evolve and Bionavigator).

Subsequent data analysis was performed using Genespring (Agilent). For each sample the per spot average of the signals in the 3 replicates was calculated, resulting in a data set containing 144 peptide signals for each of the 8 samples. The signals were normalized per peptide by dividing all signals by the mean of all signals on that particular peptide as measured in all the other samples (both normal and RCC kidney samples). 97 features were subsequently selected based on CV levels below 40% in at least one condition. The peptides and their normalized values were used in a clustering analysis and visualized in a heatmap representation. Using univariate analysis a student t-Test was used to identify peptides significantly up or down regulated in the tumor tissue versus the paired normal, non-tumor kidney tissue obtained from the same patient.

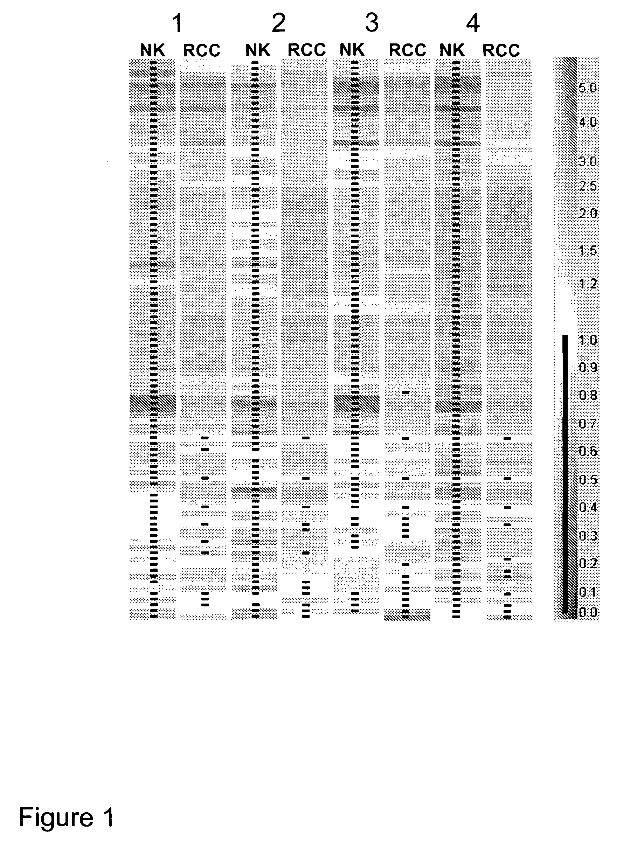

The data analysis of the inhibition experiments with sorafinib or sunitinib involved calculations of the degree of inhibition by the inhibitor per peptide. Therefore the signal obtained in the presence of the inhibitor was divided by the signal obtained in the absence of the inhibitor. The data were represented in a heatmap. A heatmap showing the ratio of inhibited versus not inhibited phosphorylation signal in each of the peptide markers and some other peptide markers listed in Table 1, is provided in Figure 1. Whereas the non-dotted squares indicate a positive ratio and the dotted squares a negative ratio, the grey intensity of the squares is proportional to the ratio level.

Figure 1 shows the results obtained by comparing the peptide phosphorylation levels resulting from peptide microarray incubation with normal kidney (NK) versus tumor kidney (RCC) tissue for 4 patients. The majority of the peptides showed enhanced phosphorylation levels. The kinase activity was found to be higher in the tumor tissue compared to normal tissue.

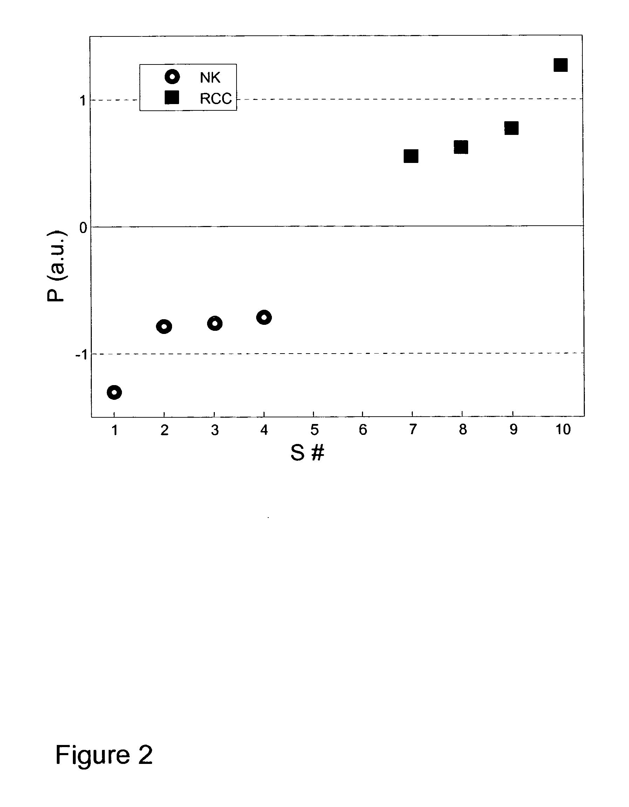

In an additional analysis using the 44 peptide markers listed in Table 1 with SEQ ID NO 1 to 44, PLS (partial least square) methodology was used on the normal and tumor (RCC) kidney samples, as shown in Figure 2. On the Y-axis the value of the classifier (P) is represented, whereas the X-axis represents the samples ranked by the classifier value. This classifier classifies the tissue as RCC if the value is above 0, and as normal tissue if the values is below 0. When compared to the classification by standard pathology (indicated by the shape of the data points presented) the samples for which a value above 0 was measured in the kinase activity profiling test, were all RCC samples. The samples for which a value below 0 was measured in the kinase activity profiling test, were all normal kidney tissue samples.

It can therefore be concluded that kinase activities of individual RCC specimens can be measured in an accurate way.

### EXAMPLE 2 - Example showing (sub)typing of RCC

The present example illustrates to what extend the peptides show enhanced phosphorylation in each patient an on how these phosphorylation profiles can be used for (sub)typing said RCC.

The results obtained from the clustering analysis as provided in example 1 were further interpreted. A further interpretation of the results as provided in example 1, revealed the insight that the RCC samples might by discerned into two subtypes.

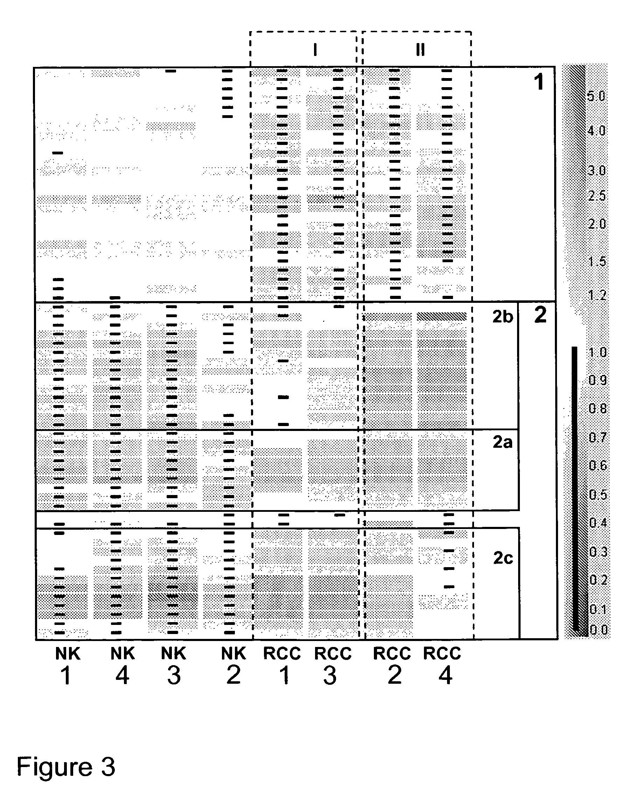

The peptide set number 1, as indicated in figure 3 (set 1) and the peptide set 2 (set 2) clearly discriminate the normal (NK) from the tumor tissue (RCC).

Peptide set 2 can be further divided into three separate subsets: subset 2a (see table 2a) shows increased phosphorylation in all 4 RCC samples, subset 2b (see table 2b) shows increased phosphorylation in especially sample 2 and 4, but hardly in the other samples and subset 2c (table 2c) shows increased phosphorylation in especially sample 1 and 3, but hardly in the other samples. As a result the profiles of tumor sample 1 and 3 and the profiles of tumor sample 2 and 4, can be regarded as stereotypical profiles for tumor subtype I and II, respectively (figure 3). Especially of the peptides in subset 2b and 2c are discriminative in subdividing these types of RCC.

| Table 2a | | |
|---|---|---|
| **SEQ ID NO** | **Peptide marker Name** | **Peptide marker Sequence** |
| 40 | PGFRB_709_721 | RPPSAELYSNALP |
| 25 | PDPK1_2_14 | ARTTSQLYDAVPI |
| 12 | EPHA7_607_619 | TYIDPETYEDPNR |
| 30 | EPHA1_774_786 | LDDFDGTYETQGG |
| 6 | EPHB1_771_783 | DDTSDPTYTSSLG |
| 2 | EPHA2_765_777 | EDDPEATYTTSGG |
| 33 | PDPK1_369_381 | DEDCYGNYDNLLS |
| 45 | CTNB1_79_91 | VADIDGQYAMTRA |
| 13 | RON_1346_1358 | SALLGDHYVQLPA |

| Table 2b | | |
|---|---|---|
| **SEQ ID NO** | **Peptide marker Name** | **Peptide marker Sequence** |
| 46 | PGFRB_771_783 | YMAPYDNYVPSAP |
| 29 | ENOG_37_49 | SGASTGIYEALEL |
| 3 | JAK2_563_577 | VRREVGDYGQLHETE |
| 7 | P85A_600_612 | NENTEDQYSLVED |
| 15 | SRC8_CHICK_492_504 | YQAEENTYDEYEN |
| 21 | PGFRB_572_584 | VSSDGHEYIYVDP |
| 27 | PECA1_706_718 | KKDTETVYSEVRK |
| 18 | FRK_380_392 | KVDNEDIYESRHE |
| 4 | PAXI_111_123 | VGEEEHVYSFPNK |
| 28 | SRC8_CHICK_476_488 | EYEPETVYEVAGA |
| 24 | FES_706_718 | REEADGVYAASGG |
| 5 | FER_707_719 | RQEDGGVYSSSGL |
| 16 | EPOR_361_373 | SEHAQDTYLVLDK |
| 17 | CD79A_181_193 | EYEDENLYEGLNL |

| Table 2c | | |
|---|---|---|
| **SEQ ID NO** | **Peptide marker Name** | **Peptide marker Sequence** |
| 35 | VGFR1_1326_1338 | DYNSVVLYSTPPI |
| 32 | PAXI_24_36 | FLSEETPYSYPTG |
| 36 | ERBB2_870_882 | LDIDETEYHADGG |
| 34 | K2C6B_53_65 | GAGFGSRSLYGLG |
| 26 | ANXA1_14_26 | IENEEQEYVQTVK |
| 20 | FAK2_572_584 | RYIEDEDYYKASV |
| 19 | VGFR1_1235_1247 | ATSMFDDYQGDSS |
| 10 | PGFRB_1014_1028 | PNEGDNDYIIPLPDP |
| 11 | LAT_194_206 | MESIDDYVNVPES |
| 14 | LAT_249_261 | EEGAPDYENLQEL |
| 1 | TNNT1_2_14 | SDTEEQEYEEEQP |
| 9 | CBL_693_705 | EGEEDTEYMTPSS |
| 8 | FGFR2_762_774 | TLTTNEEYLDLSQ |
| 38 | JAK1_1015_1027 | AIETDKEYYTVKD |

It can therefore be concluded that peptides with SEQ ID NO 1 to 44 as provided in Table 1 were shown to significantly distinguish between normal tissue and RCC tumor tissue. Furthermore, these 44 peptides can further be divided into separate subsets being discriminative for different types of RCC.

### EXAMPLE 3 - Example showing ratio inhibition by sorafinib and/or by sunitinib

The present example illustrates how protein kinase inhibitors such as sorafinib and sunitinib can be used in the method of the present invention to predict the response of a patient diagnosed with renal cell carcinoma to said compounds.

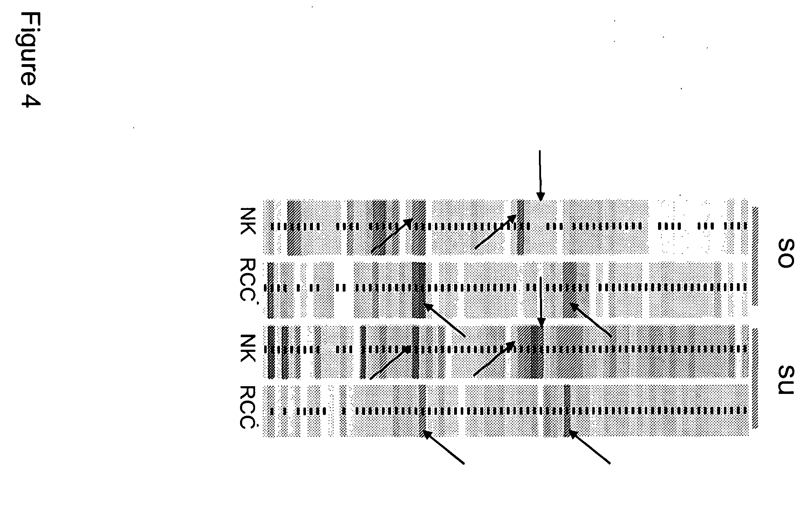

Figure 4 shows the results obtained from experiments where lysates from normal kidney tissue (NK) and tumor kidney (RCC) tissue of one particular patient were treated with a kinase inhibitor drug. Prior to performing the method according to Example 1, either sorafinib (so) or sunitinib (su) was pipetted into the lysate. The data were generated as described in example 1, and are represented as a heatmap in Figure 4. Compared to the control (DMSO) the kinase inhibitor caused a decreased phosphorylation pattern as indicated by the dotted squares. The grey intensity of the squares is proportional to the ratio level; a darker grey colour indicates a decreased phosphorylation of that peptide (lower ratio of signal in presence versus signal in absence of the inhibitor) versus control. The non-dotted squares indicate an increased phosphorylation versus control.

It is clear that drug effects can be monitored using this method. The drug affects the degree of inhibition or potency and the selectivity (more peptide inhibition is caused by larger effect of the drug on the kinases and therefore the drug is less selective). Also an increased peptide inhibition would lead to a larger amount of normal tissues being affected by the drug, making the drug less tumor tissue specific.

It can be concluded from the results in figure 4 that the profiles of sunitinib and sorafinib in tumor tissue from the same patient are similar (e.g. indicated peptides are highly inhibited in by both on-chip treatments). This is exemplified by the peptides indicated by downwards arrows, in both the sorafinib and in the sunitinib treatment these peptides are highly inhibited in comparison to other peptides.

It can further be concluded from the results in figure 4 that the profiles of sunitinib and sorafinib in normal tissue from the same patient are similar when particular peptide phosphorylation inhibition events are monitored. E.g. peptides indicated by upward arrows show inhibition in both on-chip treatments. These are candidate markers for off-target and thus adverse side effects. But other peptides show clear difference, e.g. peptides indicated by horizontal arrows show inhibition with sunitinib (su) but not with sorafinib (so) treatment. This is a marker that could differentiate off-target and thus adverse site effects found in actually treating the RCC patient with sunitinib, but not found when the patient is treated with sorafinib.

## Claims

1. A method for diagnosing renal cell carcinoma in a subject, comprising the steps of:
(a) measuring kinase activity of a kidney lysate sample from said subject, by contacting said sample with at least one protein kinase substrate, thereby providing a phosphorylation profile of said kidney sample, and,
(b) determining from said phosphorylation profile the presence or absence of renal cell carcinoma in said subject, thereby diagnosing renal cell carcinoma in said subject.

2. Method according to Claim 1, wherein said phosphorylation profile comprises the phosphorylation levels of phosphorylation sites present in any of the peptide markers as listed in Table 1 with SEQ ID NO 1 to 44.

3. Method according to Claims 1 or 2, wherein said kinase activity of said kidney lysate sample from said subject is measured in the presence and in the absence of a protein kinase inhibitor, thereby providing a phosphorylation profile of said sample in the presence and in the absence of a protein kinase inhibitor, and, determining from said phosphorylation profiles in the presence and in the absence of a protein kinase inhibitor the differential phosphorylation level, said differential phosphorylation level indicating the presence or absence of renal cell carcinoma in said subject.

4. Method according to any of Claims 1 to 3, additionally establishing from said phosphorylation profile(s) of said sample a classifier parameter, said classifier parameter determining the presence or absence of renal cell carcinoma in said subject.

5. Method according to any of Claims 1 to 4, wherein said phosphorylation profile or said classifier parameter indicates the presence or absence of renal cell carcinoma in said subject or provides an undetermined diagnosis.

6. The method according to any of Claims 2 to 5, wherein said peptide markers are at least two of the peptide markers selected from the group consisting of the peptide markers with any of SEQ ID NO 1 to 44.

7. The method according to any of Claims 3 to 6, wherein said protein kinase inhibitor is Sorafenib or Sunitinib.

8. The method according to any of claims 2 to 7, wherein said phosphorylation sites are present on proteins, peptides or peptide mimetics immobilized on a solid support, and preferably a porous solid support.

9. The method according to Claim 8, wherein said peptides are any of the peptides as listed in Table 1 with SEQ ID NO 1 to 44.

10. A method for predicting the response of a patient diagnosed with renal cell carcinoma to a medicament, wherein the kinase activity of a kidney lysate sample, obtained from the renal cell carcinoma from said patient, is measured in the presence and in the absence of said medicament and wherein said kinase activity in the presence of said medicament is compared to the kinase activity in the absence of said medicament thereby determining the response of said patient to said medicament, wherein said kinase activity measurement provides phosphorylation profiles of said sample in the presence and in the absence of said medicament.

11. The method according Claim 10, wherein said phosphorylation profiles comprise the phosphorylation levels of phosphorylation sites present in any of the peptide markers as listed in Table 1.

12. A computer program product adapted to perform the methods of any one of claims 1 to 11 and 13.

13. A method for typing or subtyping renal cell carcinoma, comprising the steps of:
(a) measuring the kinase activity of a renal cell carcinoma sample according to the method of Claims 1 to 9, thereby providing a phosphorylation profile of said renal cell carcinoma sample, and,
(b) determining from said phosphorylation profile the type or subtype of renal cell carcinoma.

## Patentansprüche

1. Verfahren zur Diagnose eines Nierenzellkarzinoms bei einem Individuum, wobei man in den Schritten
(a) Kinaseaktivität einer Nierenlysatprobe aus dem Individuum misst, indem man die Probe mit wenigstens einem Proteinkinasesubstrat in Kontakt bringt, womit ein Phosphorylierungsprofil der Nierenprobe bereitgestellt wird, und
(b) anhand des Phosphorylierungsprofils das Vorhandensein oder Nichtvorhandensein eines Nierenzellkarzinoms bei dem Individuum bestimmt, womit die Diagnose eines Nierenzellkarzinoms bei dem Individuum erfolgt.

2. Verfahren nach Anspruch 1, wobei das Phosphorylierungsprofil die Phosphorylierungsniveaus von in einem der Peptidmarker gemäß Tabelle 1 mit SEQ ID NO 1 bis 44 vorliegenden Phosphorylierungsstellen umfasst.

3. Verfahren nach Anspruch 1 oder 2, wobei die Kinaseaktivität der Nierenlysatprobe aus dem Individuum in Gegenwart und in Abwesenheit eines Proteinkinase-Inhibitors gemessen wird, womit ein Phosphorylierungsprofil der Probe in Gegenwart und in Abwesenheit eines Proteinkinase-Inhibitors bereitgestellt und anhand der Phosphorylierungsprofile in Gegenwart und in Abwesenheit eines Proteinkinase-Inhibitors das unterschiedliche Phosphorylierungsniveau bestimmt wird, wobei das unterschiedliche Phosphorylierungsniveau das Vorhandensein oder Nichtvorhandensein eines Nierenzellkarzinoms bei dem Individuum anzeigt.

4. Verfahren nach einem der Ansprüche 1 bis 3, wobei zusätzlich anhand des Phosphorylierungsprofils bzw. der Phosphorylierungsprofile der Probe ein Klassifizierungsparameter festgelegt wird, wobei der Klassifizierungsparameter das Vorhandensein oder Nichtvorhandensein eines Nierenzellkarzinoms bei dem Individuum bestimmt.

5. Verfahren nach einem der Ansprüche 1 bis 4, wobei das Phosphorylierungsprofil bzw. der Klassifizierungsparameter das Vorhandensein oder Nichtvorhandensein eines Nierenzellkarzinoms bei dem Individuum anzeigt oder eine unbestimmte Diagnose liefert.

6. Verfahren nach einem der Ansprüche 2 bis 5, wobei es sich bei den Peptidmarkern um wenigstens zwei der aus der aus den Peptidmarkern mit einer der SEQ ID NO 1 bis 44 bestehenden Gruppe ausgewählten Peptidmarker handelt.

7. Verfahren nach einem der Ansprüche 3 bis 6, wobei es sich bei dem Proteinkinase-Inhibitor um Sorafenib oder Sunitinib handelt.

8. Verfahren nach einem der Ansprüche 2 bis 7, wobei die Phosphorylierungsstellen auf auf einem festen Träger und vorzugsweise auf einem porösen festen Träger immobilisierten Proteinen, Peptiden oder Peptidemimetika vorliegen.

9. Verfahren nach Anspruch 8, wobei es sich bei den Peptiden um beliebige Peptide gemäß Tabelle 1 mit SEQ ID NO 1 bis 44 handelt.

10. Verfahren zur Vorhersage des Ansprechens eines mit Nierenzellkarzinom diagnostizierten Patienten auf ein Arzneimittel, wobei die Kinaseaktivität einer aus dem Nierenzellkarzinom von dem Patienten erhaltenen Nierenlysatprobe in Gegenwart und in Abwesenheit des Arzneimittels gemessen wird und wobei die Kinaseaktivität in Gegenwart des Arzneimittels mit der Kinaseaktivität in Abwesenheit des Arzneimittels verglichen wird, womit das Ansprechen des Patienten auf das Arzneimittel bestimmt wird, wobei durch die Kinaseaktivitätsmessung Phosphorylierungsprofile der Probe in Gegenwart und in Abwesenheit des Arzneimittels bereitgestellt werden.

11. Verfahren nach Anspruch 10, wobei die Phosphorylierungsprofile die Phosphorylierungsniveaus von in einem der Peptidmarker gemäß Tabelle 1 vorliegenden Phosphorylierungsstellen umfassen.

12. Computerprogrammprodukt, angepasst zur Ausführung der Verfahren gemäß einem der Ansprüche 1 bis 11 und 13.

13. Verfahren zur Typisierung oder Subtypisierung eines Nierenzellkarzinoms, wobei man in den Schritten
(a) die Kinaseaktivität einer Nierenzellkarzinomprobe nach dem Verfahren gemäß Anspruch 1 bis 9 misst, womit ein Phosphorylierungsprofil der Nierenzellkarzinomprobe bereitgestellt wird, und
(b) anhand des Phosphorylierungsprofils den Typ oder Subtyp des Nierenzellkarzinoms bestimmt.

## Revendications

1. Procédé de diagnostic d'un carcinome à cellules rénales chez un sujet, comprenant les étapes de :
(a) mesure de l'activité kinase d'un échantillon de lysat de rein dudit sujet, par mise en contact dudit échantillon avec au moins un substrat de protéine kinase, de manière à obtenir un profil de phosphorylation dudit échantillon de rein, et,
(b) détermination à partir dudit profil de phosphorylation de la présence ou l'absence de carcinome à cellules rénales chez ledit sujet, de manière à diagnostiquer un carcinome à cellules rénales chez ledit sujet.

2. Procédé selon la revendication 1, dans lequel ledit profil de phosphorylation comprend les taux de phosphorylation de sites de phosphorylation présents dans l'un quelconque des marqueurs peptidiques répertoriés dans le tableau 1 avec SEQ ID NO 1 à 44.

3. Procédé selon les revendications 1 ou 2, dans lequel ladite activité kinase dudit échantillon de lysat de rein dudit sujet est mesurée en présence et en l'absence d'un inhibiteur de protéine kinase, de manière à obtenir un profil de phosphorylation dudit échantillon en présence et en l'absence d'un inhibiteur de protéine kinase, et, déterminer à partir desdits profils de phosphorylation en présence et en l'absence d'un inhibiteur de protéine kinase le taux de phosphorylation différentiel, ledit taux de phosphorylation différentiel indiquant la présence ou l'absence d'un carcinome à cellules rénales chez ledit sujet.

4. Procédé selon l'une quelconque des revendications 1 à 3, établissant en outre à partir dudit/desdits profil(s) de phosphorylation dudit échantillon, un paramètre de classification, ledit paramètre de classification déterminant la présence ou l'absence d'un carcinome à cellules rénales chez ledit sujet.

5. Procédé selon l'une quelconque des revendications 1 à 4, dans lequel ledit profil de phosphorylation ou ledit paramètre de classification indique la présence ou l'absence d'un carcinome à cellules rénales chez ledit sujet ou produit un diagnostic indéterminé.

6. Procédé selon l'une quelconque des revendications 2 à 5, dans lequel lesdits marqueurs peptidiques sont au moins deux des marqueurs peptidiques choisis dans le groupe constitué des marqueurs peptidiques ayant l'un quelconque de SEQ ID NO 1 à 44.

7. Procédé selon l'une quelconque des revendications 3 à 6, dans lequel ledit inhibiteur de protéine kinase est le sorafénib ou le sunitinib.

8. Procédé selon l'une quelconque des revendications 2 à 7, dans lequel lesdits sites de phosphorylation sont présents sur des protéines, des peptides ou des mimétiques de peptide immobilisés sur un support solide, et de préférence un support solide poreux.

9. Procédé selon la revendication 8, dans lequel lesdits peptides sont l'un quelconque des peptides répertoriés dans le tableau 1 avec SEQ ID NO 1 à 44.

10. Procédé pour prédire la réponse d'un patient diagnostiqué avec un carcinome à cellules rénales à un médicament, dans lequel l'activité kinase d'un échantillon de lysat de rein, obtenu à partir du carcinome à cellules rénales dudit patient, est mesurée en présence et en l'absence dudit médicament et dans lequel ladite activité kinase en présence dudit médicament est comparée à l'activité kinase en l'absence dudit médicament de manière à déterminer la réponse dudit patient audit médicament, ladite mesure d'activité kinase produisant des profils de phosphorylation dudit échantillon en présence et en l'absence dudit médicament.

11. Procédé selon la revendication 10, dans lequel lesdits profils de phosphorylation comprennent les taux de sites de phosphorylation présents dans l'un quelconque des marqueurs peptidiques répertoriés dans le tableau 1.

12. Produit de programme informatique adapté pour conduire les procédés de l'une quelconque des revendications 1 à 11 et 13.

13. Procédé de typage ou de sous-typage de carcinome à cellules rénales, comprenant les étapes de :
(a) mesure de l'activité kinase d'un échantillon de carcinome à cellules rénales selon le procédé des revendications 1 à 9, de manière à produire un profil de phosphorylation dudit échantillon de carcinome à cellules rénales, et,
(b) détermination à partir dudit profil de phosphorylation du type ou sous-type de carcinome à cellules rénales.
